# FASCICULE DE BREVET EUROPEEN

(11) **EP 2 696 844 B1**
(45) Date de publication et mention de la délivrance du brevet: **13.11.2019**
(21) Numéro de dépôt: 12713160.5
(22) Date de dépôt: 11.04.2012
(51) Int. Cl.: A61K 8/49, A61K 31/522, A61K 45/06, A61Q 19/00

(54) **COMPOSES ACTIVATEURS DES PEPTIDYL-ARGININE DESIMINASES 1 ET/OU 3 DANS L'EPIDERME ET LEURS UTILISATIONS**
PEPTIDYLARGININDEIMINASE-1- UND/ODER 3-AKTIVATORVERBINDUNGEN IN DER EPIDERMIS UND IHRE VERWENDUNG
PEPTIDYL ARGININE DEIMINASE 1 AND/OR 3 ACTIVATOR COMPOUNDS IN THE EPIDERMIS AND USES THEREOF

(30) Priorité: 11.04.2011 FR 1153135
(43) Date de publication de la demande: 19.02.2014
(73) Titulaire: Pierre Fabre Dermo-Cosmétique, 92100 Boulogne-Billancourt (FR)
(72) Inventeur: DUPLAN, Hélène, F-31320 Auzeville Tolosane (FR); DAUNES-MARION, Sylvie, F-31000 Toulouse (FR); POIGNY, Stéphane, F-31600 Saubens (FR); MECHIN, Marie-Claire, F-31180 Lapeyrousse-Fossat (FR); SERRE, Guy, F-31100 Toulouse (FR); SIMON, Michel, F-31450 Belberaud (FR)
(74) Mandataire: Regimbeau
(86) Numéro de dépôt international: PCT/EP2012/056596
(87) Numéro de publication internationale: WO 2012/140095

(56) Documents cités:
- EP-A1- 1 181 926
- WO-A1-2007/032624
- WO-A2-2004/041168
- WO-A2-2010/019450
- GB-A- 2 221 391
- US-A1- 2002 150 597

## Description

La présente invention concerne le domaine des molécules capables d'activer la peptidyl-arginine désiminase de type I (ou PAD1) et/ou la peptidyl-arginine désiminase de type III (ou PAD3) dont l'acéfylline ou un de ses sels ; et toutes les applications de ces molécules dans les domaines de la cosmétique et de la thérapeutique.

La présente invention concerne l'utilisation cosmétique de l'acéfylline ou un de ses sels comme agent hydratant pour la peau.

La peau est formée de trois compartiments, l'hypoderme, le plus profond, le derme et l'épiderme. Ce dernier est un épithélium malpighien cornifié qui protège le corps des agressions mécaniques, chimiques et biologiques, empêche les pertes hydriques en limitant l'évaporation de l'eau contenue dans la peau et participe à la photo-protection en adsorbant une partie des rayons ultra-violets. Ces fonctions vitales sont appelées collectivement «fonctions de barrière épidermique».

L'épiderme est constitué majoritairement de kératinocytes. Ceux-ci prolifèrent au niveau de la couche basale puis subissent un programme de différenciation vectorisé pour constituer successivement les couches épineuse puis granuleuse. Finalement, au cours de la cornification, ils meurent et se transforment en cornéocytes. L'accumulation des cornéocytes forme la couche cellulaire la plus externe de l'épiderme, appelée couche cornée ou *stratum corneum.* Le *stratum corneum* est le principal responsable des fonctions de barrière épidermique, en raison de sa résistance mécanique, de son étanchéité et de son contenu en peptides antimicrobiens et en acide urocanique.

Les cornéocytes sont dépourvus de noyau et des autres organelles cellulaires. Ils sont formés d'une matrice fibreuse, contenant principalement des kératines et de la filaggrine, entourée par une coque protéique résistante qui a remplacé la membrane plasmique, l'enveloppe cornée. Les cornéocytes sont liés les uns aux autres par des structures jonctionnelles originales, les cornéodesmosomes. Au cours de la desquamation, processus très finement contrôlé par de nombreuses protéases et leurs inhibiteurs, les cornéocytes les plus superficiels se détachent de la peau après protéolyse des cornéodesmosomes.

Physiologiquement, la couche cornée contient 10 à 15% d'eau. Cette hydratation qui doit être maintenue quelles que soient les conditions hydriques extérieures, est essentielle à la barrière épidermique et à la desquamation. En effet, elle permet l'activité des enzymes qui sont nombreuses, tant à l'intérieur (protéases, transglutaminases, PADs, etc.) qu'à l'extérieur (protéases, lipases, glycosydases, etc.) des cornéocytes. Elle a aussi un effet plastifiant permettant au *stratum corneum* de conserver son élasticité et son intégrité après un stress mécanique.

Une diminution de l'hydratation de la couche cornée, caractéristique de la peau « sèche » ou xérose, se manifeste par une sensation de tiraillement, un toucher déplaisant, un aspect écailleux et l'apparition de craquelures et de squames persistantes en surface. Au niveau moléculaire, elle induit a) une diminution de la dégradation des composants desmosomaux (cornéodesmosine, cadhérines desmosomales et protéines de la plaque) et la rétention des cornéodesmosomes sur toute la surface cornéocytaire et ceci sur toute la hauteur de la couche cornée, conduisant à une hyperkératose et b) des changements dans la maturation des lipides inter-cellulaires et des enveloppes cornées.

La xérose peut survenir sur tout territoire anatomique et dans des contextes très variés, par exemple dans certaines conditions climatiques (froid, vent, sécheresse, passage rapide et répété d'un bâtiment climatisé où l'air est froid et sec, à l'extérieur où il est chaud et humide), sous l'effet de stress psychologiques et de facteurs chimiques (alcool, solvants organiques, détergents, etc. ; par exemple les lavages répétés avec certains savons) ou physiques (rayonnements ultra-violets). Elle peut aussi apparaître chez les nouveau-nés comme conséquence du passage brutal d'un milieu aqueux - le liquide amniotique - au milieu aérien et chez les personnes âgées, en fonction de la saison. Mais elle peut aussi être induite par l'exposition au soleil.

De nombreuses maladies de peau se traduisent également par une perturbation de la barrière épidermique et une sécheresse de la couche cornée : les ichtyoses et en particulier l'ichtyose vulgaire (OMIM 146700), la dermatite atopique (OMIM 605803), le psoriasis (OMIM 177900).

L'hydratation de la couche cornée est assurée par le facteur naturel d'hydratation (FNH) qui permet aux couches de cornéocytes les plus externes de retenir l'humidité en s'opposant à l'action desséchante de l'environnement. La composition du FNH qui peut représenter jusqu'à 20% du poids sec de la couche cornée est donnée ci-dessous :

| | % |
|---|---|
| Acides aminés libres et dérivés | 52,0 |
| *(Dont acide pyrrolidone carboxilique* | *12,0)* |
| Lactate | 12,0 |
| Sucres, acides organiques, peptides | 8,5 |
| Urée | 7,0 |
| Chlorure | 6,0 |
| Sodium | 5,0 |
| Potassium | 4,0 |
| Calcium, magnésium, phosphate | 3,5 |
| Ammoniaque, acide urique, glucosamine | 1,5 |
| Citrate, formate | 0,5 |

Plus de 50% de ces constituants correspondent à des acides aminés libres et à certains de leurs dérivés. En particulier, l'acide pyrrolidone carboxylique, un dérivé spontané de la glutamine qui peut représenter jusqu'à 12 % du FNH, joue un rôle hygroscopique majeur. L'acide urocanique, formé à partir d'histidine par l'histidase, absorbe quant à lui, une partie du rayonnement ultra-violet-B.

Tous ces acides aminés sont directement issus de la dégradation de la filaggrine, protéine basique de 37 kDa, synthétisée par les kératinocytes granuleux sous forme d'un précurseur de grande taille (400 kDa), la profilaggrine. Celle-ci, composant essentiel des granules de kératohyaline, est formée par la répétition de 10 à 12 sous-unités de filaggrine (suivant les individus), longues chacune de 327 acides aminés et reliées entre elles par un peptide de liaison de 7 acides aminés. Au cours de la cornification, la profilaggrine est découpée en sous-unités basiques de filaggrine. Celles-ci s'associent aux filaments intermédiaires de kératines (K1 et K10) et facilitent leur agrégation et la formation de la matrice fibreuse intra-cornéocytaire. Plus tard dans la couche cornée, la filaggrine est désiminée ce qui entraîne sa dissociation des filaments intermédiaires. Elle peut alors être totalement dégradée par la calpaïne I, la caspase 14 et la bléomycine hydrolase, ce qui génère les acides aminés constitutifs du FNH. La désimination de la filaggrine est donc une étape indispensable, voire limitante, au maintien de l'hydratation de la couche cornée et à la fonction de barrière épidermique.

La désimination, ou citrullination, est une modification post-traductionnelle catalysée par une famille d'enzymes dépendantes du calcium, les PADs (E.C.3.5.3.15). Elle correspond à la transformation des résidus arginyl (chargés positivement) en résidus citrullyl (neutres). Il existe cinq isotypes de PADs, codés par cinq gènes distincts (nommés *PADI*), regroupés sur le bras court du chromosome 1 humain au locus 1p35-36. Ce sont les PAD1, PAD2, PAD3, PAD4 et PAD6. Alors que PAD2 est ubiquiste, les autres isotypes sont exprimées de façon plus restreinte en fonction du tissu analysé. En particulier, seules les PAD1, PAD2 et PAD3 sont détectées dans l'épiderme humain normal. Sur la base d'arguments biochimiques et physico-chimiques et parce qu'elles sont co-localisées avec la filaggrine dans la matrice fibreuse intra-cornéocytaire, il a été démontré que PAD1 et PAD3 sont les isotypes responsables de la désimination de la filaggrine. Ce sont donc ces isotypes qu'il faut viser si l'on veut agir sur la production des acides aminés du FNH.

La production du FNH est d'ailleurs régulée via le catabolisme de la filaggrine, en fonction du taux d'humidité extérieur, selon un mécanisme adaptatif. Par exemple, durant les derniers jours de développement embryonnaire du rat, la filaggrine s'accumule sur toute la hauteur de la couche cornée. Quelques heures après la naissance, elle est protéolysée dans la partie externe, selon un profil identique à celui observé dans la peau des adultes. Le maintien des rats nouveau-nés en humidité relative supérieure à 80% prévient le déclenchement de ce processus de dégradation, sans mettre en jeu la synthèse de la protéine. De même, le taux des acides aminés libres et la conductance à la surface de la peau (reflets de l'hydratation) sont rétablis en 3 jours quand des souris sans poils (dites « hairless ») sont transférées d'un environnement humide à un environnement normal.

Pour réduire la sécheresse cutanée, améliorer l'hydratation de la couche cornée et traiter la xérose ou les ichtyoses, des applications topiques sur la peau de formulations cosmétiques ou pharmaceutiques présentant des actifs hygroscopiques sont utilisées. Ces formulations contiennent par exemple de l'urée ou de l'acide lactique, autres composants du FNH. De façon générale les hydroxy-acides sont devenus l'une des plus importantes classes de composés pour l'industrie cosmétique, à cause de leurs propriétés hydratantes et « anti-âge » mais l'hydratant le plus populaire et le plus couramment utilisé est certainement le glycérol.

Quelles que soient les formulations déjà développées, il est évidemment nécessaire de proposer de nouveaux actifs utiles à l'industrie cosmétique et/ou à la pharmacopée pour améliorer les symptômes liés à la sécheresse cutanée. Le but de la présente invention est justement de favoriser la production du FNH en utilisant de nouveaux composés capables d'agir sur les PADs 1 et 3 pour favoriser l'hydratation naturelle de la couche cornée. Ceci peut trouver une application particulièrement avantageuse dans le domaine des produits de soin et/ou de maquillage de la peau du corps ou du visage, des lèvres, des cils, des sourcils, des cheveux, du cuir chevelu ou des ongles; d'un produit solaire ou autobronzant; d'un produit capillaire notamment de coloration, de conditionnement et/ou de soin des cheveux.

En effet, après d'importantes recherches, la Demanderesse a découvert de façon surprenante et inattendue que l'acéfylline ou un de sels, la caféine et la théobromine, utilisées seules ou en combinaison, ont la faculté d'augmenter l'activité des PAD1 et/ou PAD3, en particulier leur capacité à désiminer leur substrat physiologique la filaggrine, pour favoriser l'hydratation naturelle de la couche cornée. La formule chimique de ces molécules est précisée ci-après : 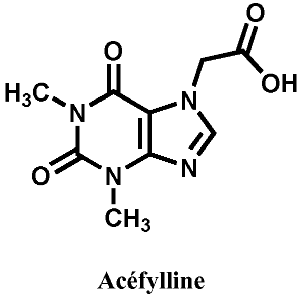 2-(1,3-dimethyl-2,6-dioxo-2,3-dihydro-1*H*-purin-7(6*H*)-yl)acetic acid 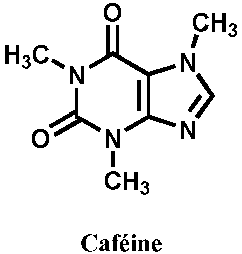 1,3,7-trimethyl-1*H*-purine-2,6(3*H*,7*H*)-dione 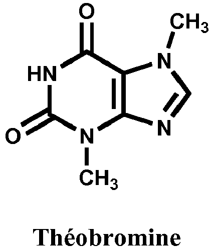 3,7-dimethyl-1*H*-purine-2,6(3*H*,7*H*)-dione

Il a été proposé que la réaction de désimination se divise en 5 étapes successives :
i) attaque nucléophile du Cζ du peptidyl-L-arginine par le groupement thiol de la Cys du site actif de l'enzyme ;
ii) formation de liaisons, hydrogènes et saline, entre les Asp du site actif et le substrat ;
iii) coupure de la liaison entre le Cζ et le Nη2 du peptidyl-L-arginine et libération d'ammoniac;
iv) deuxième attaque nucléophile, cette fois-ci par une molécule d'eau et
v) hydrolyse de l'adduit formé à l'issue de la réaction précédente et libération du produit final de la désimination, le peptidyl-L-citrulline.

Ainsi, la présente invention a pour objet l'activation, par les actifs décrits précédemment, utilisés seuls ou en combinaison, de l'une ou l'autre de ces 5 étapes de la réaction catalysée par les PAD1 ou 3.

L'objet de la présente invention concerne donc l'utilisation cosmétique d'une composition comprenant de l'acéfylline comme agent hydratant de la couche cornée de la peau.
Plus particulièrement, ladite composition est destinée à favoriser la désimination de la filaggrine dans l'épiderme.
La composition selon la présente invention est également destinée à favoriser la production de FNH (facteur naturel d'hydratation) dans la couche cornée.

Ces activateurs de PAD1 et/ou PAD3 ont donc été sélectionnés pour favoriser la production du FNH dans la couche cornée ainsi que pour leur utilisation à l'hydratation de l'épiderme, en particulier de la couche cornée.

Les actifs utilisés l'invention peuvent être de toutes origines, à savoir isolés de végétaux comme le caféier ou le cacaotier, produits par des micro-organismes même s'ils ne sont pas produits de manière naturelle par lesdits organismes d'origine, ou encore obtenus par synthèse chimique.

Par « sels d'acéfylline », on entend au sens de l'invention les sels organiques ou inorganiques d'acéfylline.

Comme sels organiques utilisables selon l'invention, on peut citer ceux décrits dans l'art antérieur FR 2 639 541. De préférence il s'agira du sel de triéthanolamine.

Comme sels inorganiques d'acéfylline, on peut citer les sels de sodium, potassium ou lithium.

Dans un mode de réalisation particulier de l'invention, le sel d'acéfylline est formé par l'ajout dans la composition comprenant l'acéfylline d'une base organique ou inorganique en tant qu'agent neutralisant. De préférence la base organique est la triéthanolamine et la base inorganique est NaOH.

L'invention se rapporte également aux compositions cosmétiques contenant de l'acéfylline ou un de ses sels associée à la caféine et/ou à la théobromine, en combinaison avec au moins un excipient cosmétiquement acceptable.

Cette composition pourra être préférentiellement d'origine naturelle ou synthétique, encore préférentiellement synthétique.

De préférence l'acéfylline présente dans la composition représentera entre 0,5% et 3% en poids, du poids total de la composition dans le cas d'une composition pharmaceutique et entre 0,1% et 1% en poids, du poids total de la composition dans le cas d'une composition cosmétique.

Le composé activateur de PAD1 et/ou PAD3 utilisé sera l'acéfylline ou un de ses sels.

Dans un mode de réalisation particulier de l'invention, l'acéfylline ou un de ses sels est associée à la caféine et/ou à la théobromine.

L'acéfylline ou un de ses sels, étant donné son effet activateur sur les enzymes PAD1 et/ou PAD3, vont permettre une augmentation de la dégradation de la filaggrine au sein de la couche cornée et donc de la production des acides aminés composant le FNH, en particulier de l'acide pyrrolidone carboxylique (PCA) dérivé de la glutamine. Une augmentation de la quantité dosée de cet acide témoigne de l'activité hydratante de ces composés.

Préférentiellement, les compositions selon l'invention seront administrées par voie topique.

Préférentiellement la composition selon l'invention comprendra au moins une phase grasse liquide, qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange.

La composition selon la présente description se présentera préférentiellement sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux.

L'utilisation cosmétique selon l'invention de la composition pourra permettre l'hydratation de de la couche cornée ; pour l'amélioration de toute forme de sécheresse cutanée ; pour le renforcement des fonctions de barrière de l'épiderme.

Par hydratation de l'épiderme on entend l'amélioration ou le maintien de l'équilibre en eau de l'épiderme.

Par l'amélioration de toute forme de sécheresse cutanée on entend toute amélioration de l'hydratation de l'épiderme notamment caractérisé par un manque d'eau dans la couche cornée, un film hydrolipidique situé à la surface trop fin et qui ne protège plus la peau, le manque de sébum.

Par vieillissement cutané on entend, outre les effets sur le derme et la perte d'élasticité des tissus, un affaiblissement de la fonction barrière. Malgré une perte insensible en eau peu augmentée, l'expérience clinique a montré que les personnes âgées souffrent plus souvent de sécheresse cutanée que les personnes plus jeunes et en bonne santé. Ceci s'explique en premier lieu par une altération de la barrière lipidique. La barrière épidermique serait également plus facilement altérée et plus lente à se réparer. La technique de spectroscopie Raman a permis de démontrer que les capacités du *stratum corneum* à retenir l'eau ainsi que la quantité de FNH diminuent avec l'âge, essentiellement dans les couches les plus superficielles.

Les rayons ultra-violets B sont l'une des causes principales de la survenue des cancers de la peau en raison des dommages à l'ADN qu'ils induisent et donc de leur important pouvoir mutagène. Une grande partie d'entre eux est absorbée par la mélanine, mais la première barrière photoprotectrice est assurée par un composant du FNH dont le taux moyen est d'environ 5 µg/cm² de peau, l'acide transurocanique. C'est un écran solaire naturel relativement efficace qui a été ajouté à de nombreux produits cosmétiques dans les années soixante-dix et quatre-vingt. Dans le *stratum corneum,* l'acide transurocanique dérive, comme la majorité des acides aminés du FNH, du catabolisme de la filaggrine.

La présente description divulgue aussi l'utilisation cosmétique des actifs décrits précédemment, utilisés seuls ou en combinaisons, pour l'hydratation de l'épiderme visant à favoriser la production naturelle d'acide transurocanique.

Dans le cadre de la présente invention, diverses méthodes d'analyse biochimiques ont permis de sélectionner quelques molécules modulatrices de l'activité catalytique de PAD1 et PAD3 eu égard à leur capacité particulières à désiminer la filaggrine. Ces résultats sont illustrés sur les dessins annexés qui représentent :

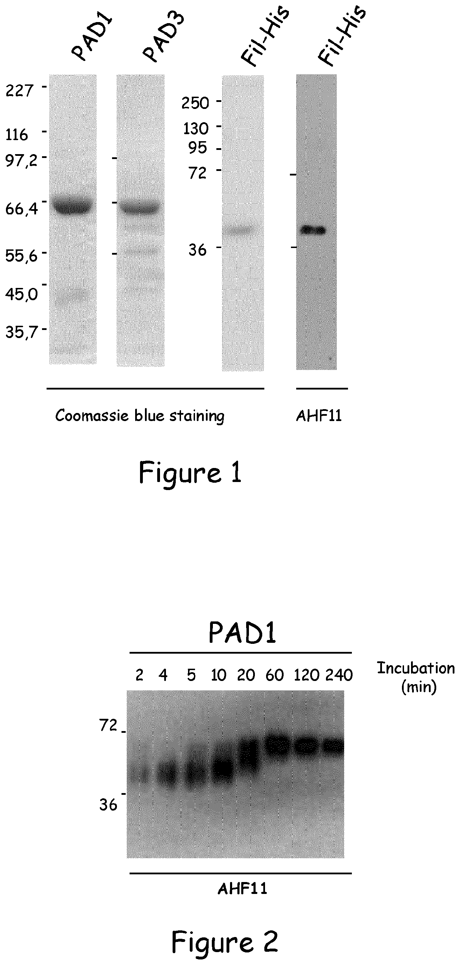

Figure 1 : Analyse des protéines recombinantes purifiées.
   Après purification, PAD1, PAD3 et la filaggrine humaine recombinante (Fil-His) ont été séparées par électrophorèse en gel de polyacrylamide en présence de SDS (PAGE-SDS), colorées au bleu de Coomassie ou immunodétectées, comme indiqué. AHF11, un anticorps monoclonal anti-filaggrine détecte la Fil-His non-désiminée vers 45 kDa. Les masses moléculaires apparentes sont indiquées à gauche en kDa.
Figure 2 : Cinétique de désimination de la filaggrine humaine recombinante (Fil-His) par PAD1.
   Après incubation avec la PAD1, la Fil-His a été immunodetéctée par AHF11, un anticorps monoclonal anti-filaggrine. La masse moléculaire apparente est indiquée à gauche en kDa et les temps d'incubation sont indiqués en minute (min) au dessus de l'illustration. On note que la désimination de la Fil-His par PAD1 induit un changement progressif de sa migration de 45 vers 66 kDa.

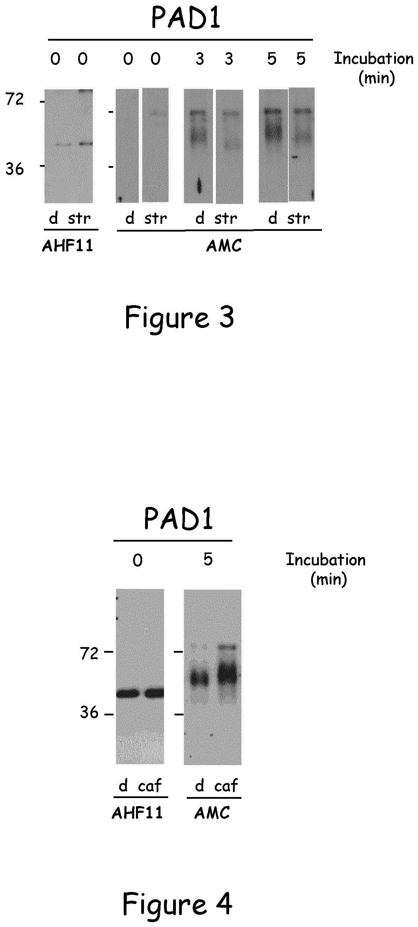

Figure 3 : Désimination de la filaggrine humaine recombinante (Fil-His) par PAD1 en présence ou en absence de streptomycine.
   Après incubation avec PAD1, en présence de 5 mM de streptomycine (str) ou en présence de 1 % de diméthylsulfoxide (d), le solvant, la Fil-His a été immunodetéctée par AHF11 un anticorps monoclonal anti-filaggrine ou par AMC, un anticorps anti-citrulline, comme indiqué. Les masses moléculaires apparentes sont indiquées à gauche en kDa et les temps d'incubation sont indiqués en minutes (min) au dessus de l'illustration. On note que PAD1 est inhibée par la streptomycine puisque l'intensité d'immunodétection par AMC est plus faible quand l'incubation a eu lieu en présence de cette molécule par rapport à une incubation réalisée en présence du solvant seul.
Figure 4 : Désimination de la filaggrine humaine recombinante (Fil-His) par PAD1 en présence ou en absence de caféine.
   Après incubation avec PAD1, en présence de 50 µM de caféine (caf) ou en présence de 1 % de diméthylsulfoxide (d), le solvant, la Fil-His a été immunodetéctée par AHF11 un anticorps monoclonal anti-filaggrine ou par AMC, un anticorps anti-citrulline, comme indiqué. Les masses moléculaires apparentes sont indiquées à gauche en kDa et les temps d'incubation sont indiqués en minutes (min) au dessus de l'illustration. On note que PAD1 est activée par la caféine puisque la Fil-His est désiminée plus fortement en présence de cette molécule qu'en présence du solvant seul.

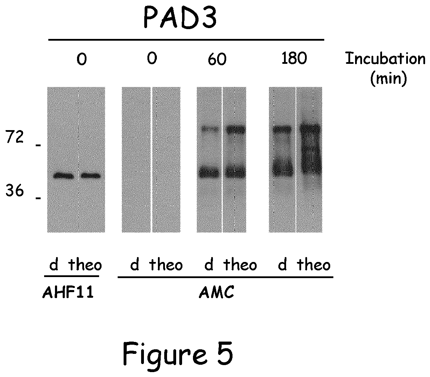

Figure 5 : Désimination de la filaggrine humaine recombinante (Fil-His) par PAD3 en présence ou en absence de théobromine.
   Après incubation avec PAD3, en présence de 200 µM de théobromine (théo) ou en présence de 1 % de diméthylsulfoxide (d), le solvant, la Fil-His a été immunodetéctée par AHF11 un anticorps monoclonal anti-filaggrine ou par AMC, un anticorps anti-citrulline, comme indiqué. Les masses moléculaires apparentes sont indiquées à gauche en kDa et les temps d'incubation sont indiqués en minutes (min) au dessus de l'illustration. On note que PAD3 est activée par la théobromine.

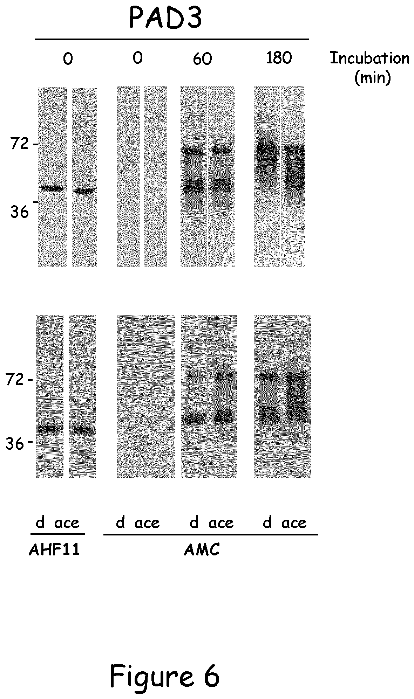

Figure 6 : Désimination de la filaggrine humaine recombinante (Fil-His) par PAD3 en présence ou en absence d'acéfylline.
   Après incubation avec PAD3, en présence d'acéfylline (acé) à la concentration finale de 50 µM (partie du haut) ou 200 µM (partie du bas), ou en présence de 1 % de diméthylsulfoxide (d), le solvant, la Fil-His a été immunodetéctée par AHF11 un anticorps monoclonal anti-filaggrine ou par AMC, un anticorps anti-citrulline, comme indiqué. Les masses moléculaires apparentes sont indiquées à gauche en kDa et les temps d'incubation sont indiqués en minutes (min) au dessus des illustrations. On note que PAD3 est activée par l'acéfylline (surtout visible à la plus forte concentration).

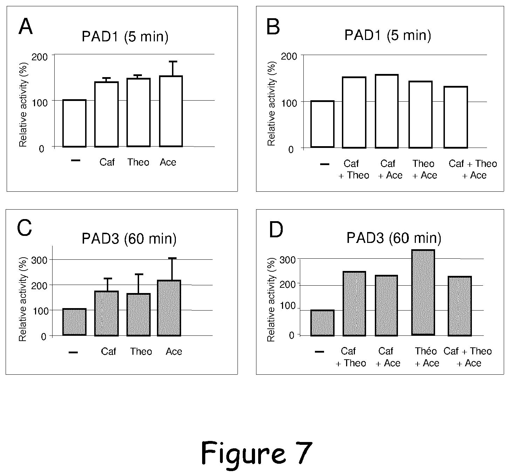

Figure 7 : Désimination de la filaggrine humaine recombinante par PAD1 ou PAD3 en présence de caféine, théobromine et/ou acéfylline, seules ou en combinaison.
   La Fil-His a été incubée en présence de PAD1 pendant 5 minutes (A-B) ou en présence de PAD3 pendant 60 minutes (C-D). Avant incubation, 312,5 µM de caféine (Caf), d'acéfylline (Acé) ou de théobromine (Théo) ont été ajoutées au mélange réactionnel, seules (A et C) ou en combinaison (B et D), comme indiqué. Après incubation, la Fil-His a été immunodetéctée par l'anticorps anti-citrulline (AMC) et l'intensité d'immunodétection a été quantifiée à l'aide du logiciel ImageJ. Les données sont reportées sous forme d'histogrammes en pourcentage relatif de l'activité de chaque enzyme par rapport aux contrôles (-) réalisés en absence d'actif.

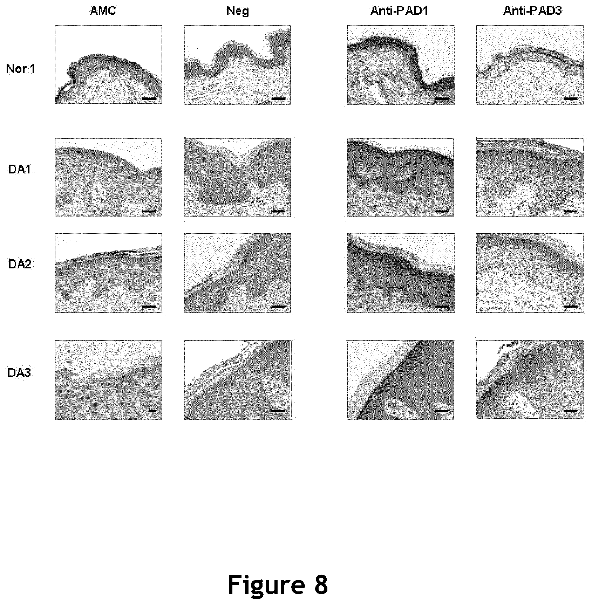

Figure 8 : PAD1, PAD3 et protéines désiminées analysées par immunohistologie sur coupes de peau humaine normale ou de peau de patients atteints de dermatite atopique.
   Les protéines désiminées ont été immunodétectées avec l'anti-citrulline (AMC) après modification chimique des citrullines. Le contrôle négatif (Neg) a été réalisé en parallèle. PAD1 et PAD3 ont été immunodétectées avec les anticorps anti-PAD1 et anti-PAD3 comme décrit précédemment (exemple 3). Des images représentatives des résultats obtenus pour huit patients (DA1, 2 et 3) et trois contrôles (Nor1) sont présentées [barre = 150 µm].

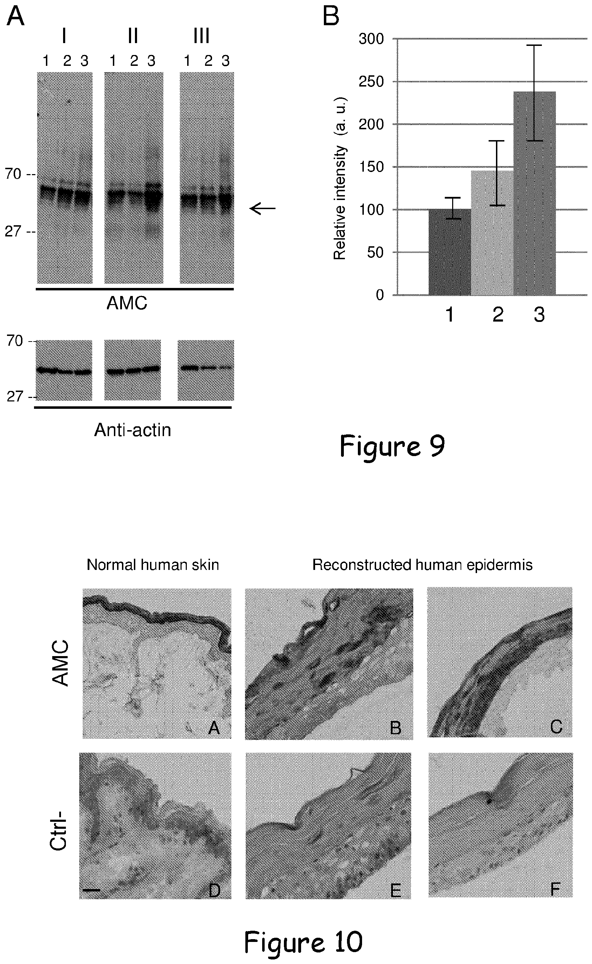

Figure 9 : Analyse par Western des protéines désiminées après l'application topique d'acéfylline sous forme de sel de triéthanolamine ou de sel de sodium, à la surface des épidermes reconstruits.
   Des épidermes reconstruits ont été traités en topique pendant 24 heures avec un gel contrôle (1) ou un gel contenant de l'acéfylline à la concentration de 3% (2 et 3) sous forme de sels de triéthanolamine (2) ou de sels de sodium (3).
   **A.** Les protéines totales ont été séparées par électrophorèse et immunodétectées avec l'anticorps anti-citrulline (AMC) et un anticorps anti-actine. L'expérience a été réalisée en triplicats (I, II et III). Les masses moléculaires apparentes sont indiquées à gauche en kDa.
   **B.** L'intensité d'immunodétection a été quantifiée à l'aide du logiciel ImageJ. Les valeurs obtenues ont été normalisées par rapport à l'actine. Elles sont reportées sous forme d'histogrammes en pourcentage relatif par rapport aux contrôles réalisés en absence d'acéfylline.
      On note que, par rapport aux épidermes traités avec le gel contrôle, l'intensité d'immunodétection des protéines citrullinées est plus importante pour les épidermes traités avec le gel contenant de l'acéfylline sous forme de sels.
Figure 10 : Analyse par immunohistologie des protéines désiminées après l'application topique d'acéfylline sous forme de sel de sodium à la surface des épidermes reconstruits.

Des cryocoupes de peau normale non traitée (A et D) et d'épidermes reconstruits traités en topique pendant 24 heures avec un gel contrôle (B et E) ou un gel contenant de l'acéfylline à la concentration de 3% sous forme de sels de sodium (C et F) ont été analysées en immunohistologie avec l'anticorps anti-citrulline (AMC) avec (A-C) ou sans (D-F ; contrôles négatifs) modification des citrullines [Barre = 150µm]. On note une augmentation de l'intensité de marquage de la couche cornée de l'épiderme reconstruit traité avec l'acéfylline sous forme de sel de sodium (C) par rapport à l'épiderme reconstruit traité avec le gel contrôle (B).

### EVALUATION PHARMACOLOGIQUE (exemples comparatifs)

### A/ Mesure de l'activité de PAD1 et/ou PAD3 à l'aide de filaggrine humaine recombinante

### Méthodes :

### 1. Production et purification de filaggrine humaine recombinante

Une sous-unité de filaggrine humaine recombinante de 324 acides aminés (numéro d'accès à la banque de données « GenBank » : AF043380) a été produite en fusion avec une étiquette de 6 histidines en COOH-terminal à l'aide du vecteur pET-41b (Merck, KGaA, Darmstardt, Germany) dans la souche *Escherichia coli* BL21 Codon plus (DE3+)-RIL (Stratagene, La Jolla, CA).

Pour ce faire, l'ADNc a tout d'abord été amplifié par PCR à l'aide du couple d'oligo-nucléotides suivant :
5'-CATATGCTATACCAGGTGAGCACTCATG-3' et
5'-CTCGAGCCCTGAACGTCCAGACCGTCC-3',
à partir d'ARNm extraits d'épiderme humain, puis purifié et cloné dans le vecteur pCRII-Topo (Invitrogen, Carlsbad, CA).

Le produit de PCR était ainsi flanqué d'un site NdeI et d'un site XhoI, ces 2 sites de restrictions additionnels permettant le sous-clonage orienté depuis le vecteur pCRII-Topo dans le vecteur d'expression pET-41b. Après vérification par séquençage, la sous-unité de filaggrine, nommée ci-après Fil-His, a été produite et purifiée par chromatographie d'affinité sur Nickel selon un protocole bien connu de l'homme de l'art. La protéine recombinante Fil-His, ainsi purifiée a été analysée par PAGE-SDS (gel 10%) et immunodétectée avec l'anticorps monoclonal anti-filaggrine AHF11. Le degré de pureté a été contrôlé par coloration au bleu de Coomassie, la protéine Fil-His présentant un poids moléculaire apparent d'environ 45 kDa (voir Figure 1). La concentration de la fraction purifiée obtenue a été mesurée (en mg/ml) à l'aide d'un NanoDrop 1000 (Fisher Scientific, Illkirch, France) en utilisant une gamme étalon d'albumine sérique bovine.

### 2. Production de PAD1 et PAD3 humaines recombinantes actives

Les PAD1 et PAD3 produites et purifiées selon les protocoles bien connus de l'homme de l'art ont été achetées auprès du Professeur Hidenari Takahara (Université d'Ibaraki, Japon). Les caractéristiques desdites PAD1 et PAD3 recombinantes humaines actives purifiées sont rappelées dans le Tableau ci-dessous :

| PAD | Activité* (unité / ml) | Concentration protéine (mg/ml) | Activité spécifique (unité/mg) |
|---|---|---|---|
| PAD1 | 206,0 | 0,696 | 296,0 |
| PAD3 | 10,2 | 0,400 | 25,5 |

| | | | |
|---|---|---|---|
| *une unité est définie comme la quantité de PAD qui catalyse la formation de 1 µmol de Bz-L-Cit-O-Et à partir de Bz-L-Arg-O-Et en 1h à 55°C. | | | |

Le degré de pureté de chaque enzyme a été contrôlé par coloration au bleu de Coomassie après séparation par PAGE-SDS (voir Figure 1).

### 3. Désimination de la Fil-His par les PAD1 et PAD3

Vingt-cinq ng de Fil-His ont été incubés à 50°C en présence de 40 mU de PAD1 ou de PAD3 dans le tampon de désimination (CaCl₂ 10 mM, dithiothréitol 5 mM, Tris-HCl 50 mM pH 7,4). Après un temps d'incubation variable selon l'isoforme de PAD (généralement 2 à 5 minutes pour PAD1 et 60 à 180 minutes pour PAD3), la réaction a été arrêtée par ajout de tampon échantillon. Les protéines ont ensuite été séparées par PAGE-SDS et immunodétectées avec l'anticorps monoclonal anti-filaggrine AHF11 et l'anticorps anti-citrulline modifiée AMC (Millipore, Mollsheim, France) dilués au 1/5000.

### Résultats :

### 1. Evaluation de la désimination de la Fil-His

Lorsque la Fil-His n'est pas désiminée sa masse moléculaire apparente en gel dénaturant est de l'ordre de 45 kDa. Elle migre sous la forme d'une bande protéique unique après coloration au bleu de Coomassie ou après immunodétection avec l'anticorps AHF11 (voir Figure 1). La désimination de la Fil-His induit une augmentation progressive de sa masse moléculaire apparente de 45 à ∼66 kDa (voir Figure 2). La forme de ∼66 kDa correspond à la forme totalement désiminée, les formes intermédiaires correspondant à des formes plus ou moins désiminées. Le degré de désimination de la Fil-His est donc corrélé à sa masse moléculaire apparente après séparation par PAGE-SDS.

La Fil-His n'est immunodétectée par l'anticorps AMC qu'après incubation avec une PAD active. L'intensité d'immunodétection est d'autant plus importante qu'elle est plus fortement désiminée (voir Figure 3).

### 2. La streptomycine inhibe la PAD1

Un changement dans la migration de la filaggrine et/ou un changement de l'intensité d'immunodétection par l'anticorps AMC permettent d'évaluer l'effet (activateur ou inhibiteur) sur les PADs d'une molécule ajoutée dans le mélange réactionnel avant le début de la réaction de désimination. L'inhibition de PAD1 par la streptomycine par exemple, a pu être mise en évidence par cette méthode (voir Figure 3). L'intensité d'immunodétection par l'anticorps AMC des bandes dont la masse est soit de ∼66 kDa, soit comprise entre 45 et ∼60 kDa, est plus faible quand la Fil-His est incubée pendant 3 ou 5 minutes avec PAD1 en présence de 5 mM de streptomycine, comparativement à une incubation en tous points identiques, en présence du solvant seul (1% de diméthylsulfoxide).

### B/ Activateurs de PAD1 et/ou de PAD3

### Méthode :

### Désimination de la Fil-His par la PAD1 ou la PAD3 en présence d'un actif ou d'une combinaison d'actifs

La désimination de la Fil-His (25 ng) a été réalisée comme décrit précédemment (paragraphe A.3), dans le tampon de désimination (CaCl₂ 10 mM, dithiothréitol 5 mM, Tris-HCl 50 mM pH 7,4) à 50°C en présence de 40 mU de PAD1 ou de PAD3, pendant 0, 3, 5, 60 et 180 minutes. L'actif en solution dans 1% de diméthylsulfoxide pour obtenir une la concentration finale de 50, 200 et 312,5 µM, ou 1% de diméthylsulfoxide seul (contrôle) ou un mélange de plusieurs actifs dilués chacun à 312,5 µM dans 1% de diméthylsulfoxide ont été ajoutés au mélange réactionnel avant addition de l'enzyme. Dans certains cas, les actifs ont été dilués dans de l'eau. Après incubation les protéines du mélange réactionnel ont été immunodétectées avec l'anticorps AMC. L'intensité d'immunodétection a été quantifiée par densitométrie avec le logiciel NIH ImageJ.

### Résultats :

### 1. Activation de la PAD1 par la caféine

Avant désimination, la Fil-His est immunodétectée par l'anticorps AHF11 comme une bande d'environ 45 kDa mais n'est pas détectée par l'anticorps AMC (voir Figure 4). Après 3 et 5 minutes d'incubation en présence de PAD1 et de diméthylsulfoxide (1% final), la Fil-His est désiminée partiellement par PAD1 : elle est alors immunodétectée par l'anticorps AMC comme une large bande de taille comprise entre 45 et ∼60 kDa. Après 3 et 5 minutes d'incubation en présence de PAD1 et de caféine (50 µM final dans 1% de diméthylsulfoxide), l'intensité d'immunodétection de cette large bande est plus importante et une bande supplémentaire, migrant vers 66 kDa, est détectée par l'anticorps AMC (voir Figure 4). Cette protéine de ∼66 kDa correspond à de la Fil-His totalement désiminée. Une analyse quantitative a confirmé ce résultat et montré une augmentation de 151% de l'intensité d'immunodétection des bandes entre 45 et ∼66 kDa après incubation en présence de caféine, comparativement au contrôle.

Des résultats comparables ont été obtenus lors d'essais réalisés avec de la caféine solubilisée et diluée dans de l'eau ultra-pure.

La caféine active donc la PAD1.

### 2. Activation de la PAD3 par la théobromine

Avant désimination, la Fil-His présente une masse apparente d'environ 45 kDa ; elle est immunodétectée par l'anticorps AHF11 mais pas par l'anticorps AMC (voir Figure 5). Après 180 minutes d'incubation en présence de PAD3 et de diméthylsulfoxide (1%), elle est désiminée partiellement par PAD3 : elle est alors immunodétectée par l'anticorps AMC comme une large bande de taille comprise entre 45 et ∼66 kDa. Après 180 minutes d'incubation en présence de PAD3 et de théobromine (200 µM dans 1% de diméthylsulfoxide), l'intensité des bandes immunodétectées par l'anticorps AMC est plus importante (voir Figure 5). La quantification de l'intensité d'immunodétection a montré une augmentation de 182%.

Des résultats comparables ont été obtenus lors d'essais réalisés avec de la théobromine solubilisée et diluée dans de l'eau ultra-pure.

La théobromine active donc la PAD3.

### 3. Activation de la PAD3 par l'acéfylline

Avant désimination, la Fil-His est immunodétectée par l'anticorps AHF11 vers 45 kDa mais n'est pas détectée par l'anticorps AMC (voir Figure 6). Après 180 minutes d'incubation en présence de PAD3 et de diméthylsulfoxide (1%), la Fil-His est désiminée partiellement par PAD1 : elle est alors immunodétectée par l'anticorps AMC comme une large bande de taille comprise entre 45 et ∼60 kDa et d'une bande de ∼66 kDa. Après 180 minutes d'incubation en présence de PAD3 et d'acéfylline (200 µM dans 1% de diméthylsulfoxide), l'intensité des bandes immunodétectées par l'anticorps AMC est nettement plus intense (voir Figure 6). La quantification de l'intensité d'immunodétection a confirmé ce résultat et montré une augmentation de 225%.

Des résultats comparables ont été obtenus lors d'essais réalisés avec de l'acéfylline solubilisée et diluée dans de l'eau ultra-pure.

L'acéfylline active donc la PAD3.

### 4. Evaluation de l'effet d'un mélange de caféine, théobromine et/ou acéfylline sur l'activité des PAD1 et PAD3

Des essais similaires de désimination de la Fil-His par PAD1 (incubations de 5 minutes) et PAD3 (incubations de 60 minutes) ont été réalisés en présence de chacun des trois actifs (caféine, théobromine et acéfylline) ou d'un mélange de deux ou trois de ces actifs. Les actifs ont été solubilisés puis dilués dans de l'eau ultra-pure. Ils ont été utilisés à la concentration finale de 312,5 µM dans le test. L'intensité d'immunodétection par l'anticorps AMC a été quantifiée comme précédemment (voir Figure 7).

Les résultats obtenus ont confirmé l'activation de PAD1 par la caféine et celle de PAD3 par la théobromine et l'acéfylline. Ils ont de plus mis en évidence que les trois actifs utilisés individuellement activent PAD1 et PAD3, avec des niveaux d'activation comparables (voir Figures 7A et 7C).

L'addition des actifs en combinaison deux à deux ou les trois ensembles n'a pas induit d'effet inhibiteur. Le même niveau d'activation de PAD1 a été observé (voir Figure 7B). L'addition de deux ou trois actifs semble même activer la PAD3 de façon plus marquée (voir Figure 7D).

La caféine, la théobromine et l'acéfylline utilisées seules ou en combinaison activent donc la PAD1 et la PAD3.

### C/ Diminution de la détection des protéines désiminées de la couche cornée de l'épiderme lésionnel de la peau des patients atteints de dermatite atopique (exemple illustratif)

### Méthodes :

### 1. Coupes de peau

Des biopsies de 3 mm de diamètre ont été effectuées en peau lésionnelle de 8 patients atteints de dermatite atopique (diagnostic réalisé par un médecin du Service de Dermatologie des Hôpitaux de Toulouse) et en peau saine de 3 contrôles, fixées au formol et incluses en paraffine. Des coupes sérielles (6 µm) ont été réalisées pour chaque biopsie, déposées sur des lames Superfrost, déparaffinées par des bains successifs de xylène et d'éthanol puis réhydratées et colorées à l'hématoxyline-éosine ou analysées par immunohistologie.

### 2. Détection des protéines désiminées par immunohistologie

Pour immunodétecter les protéines désiminées, les coupes de peau ont été incubées pendant 3 heures à 37°C dans le tampon de modification : 0,0125% de FeCl₃, 2,3 M de H₂SO₄, 1,5 M de H₃PO₄, 0,25% de diacétyl monoxime et 0,125% d'antipyrine. Des contrôles négatifs ont été réalisés systématiquement en omettant le diacétyl monoxime et l'antipyrine. Après lavage à l'eau, les coupes ont été incubées avec l'anticorps de référence AMC dilué au 1/500^{ème} puis révélées avec le coffret « Impress Reagent anti rabbit Ig PO » selon les instructions du fabricant (Vector Laboratories, Burlingame, CA) en présence de diamino-benzidine comme substrat chromogène de la peroxydase. Finalement une contre-coloration a été réalisée avec de l'hématoxyline.

### 3. Détection des PAD1 et PAD3 par immunohistologie

Les PAD1 et PAD3 ont été immunodétectées à l'aide des anticorps anti-peptides spécifiques anti-PAD1 et anti-PAD3(B3) utilisés respectivement au 1/80^{ème} et au 1/100^{ème}, selon le protocole décrit précédemment. Une contre-coloration a été réalisée avec de l'hématoxyline. Des contrôles négatifs ont été réalisés en omettant l'anticorps primaire.

### Résultats :

### 1. Diminution des protéines désiminées dans l'épiderme des patients atteints de dermatite atopique

Tous les échantillons qui n'ont pas subi la modification chimique sont négatifs. Les échantillons de peau issus des individus contrôles présentent un marquage avec l'anticorps AMC très fort et continu tout au long de la couche cornée et sur toutes les assises de cornéocytes. Par contre, les échantillons de peau des patients atteints de dermatite atopique présentent un marquage de la couche cornée nettement plus faible et souvent discontinu (voir Figure 8).

En conclusion, la désimination des protéines de l'épiderme lésionnel des patients atteints de dermatite atopique est nettement diminuée comparativement aux contrôles sains.

### 2. Expression des PAD1 et 3 dans l'épiderme des patients atteints de dermatite atopique

Pour tous les prélèvements de peaux atopiques, la PAD1 est détectée dans le cytoplasme des kératinocytes de toutes les couches vivantes de l'épiderme, avec une intensité plus forte au niveau des kératinocytes les plus différenciés. Le même profil d'immunodétection est observé pour les prélèvements de peaux normales, comme cela est bien connu de l'homme de l'art. L'intensité du marquage obtenu sur les peaux atopiques et normales est la même.

Dans l'épiderme normal, la PAD3 est détectée essentiellement dans le cytoplasme des kératinocytes granuleux, comme cela est bien connu de l'homme de l'art. Dans l'épiderme atopique de tous les patients, la PAD3 est détectée dans le cytoplasme de plusieurs assises cellulaires des kératinocytes les plus différenciés. L'intensité du marquage est le plus souvent égale à celle du marquage de l'épiderme normal (voir Figure 8).

Les PAD1 et PAD3 sont donc largement exprimées dans l'épiderme des patients atteints de dermatite atopique. Ce n'est donc pas une absence de ces enzymes qui explique la diminution de la désimination des protéines observée précédemment, mais peut-être une activité plus faible.

### D/ Augmentation des protéines désiminées de la couche cornée après traitement avec un gel contenant de l'acéfylline.

### Méthodes :

### 1. Epidermes reconstruits

Des épidermes humains reconstruits de 0,33 cm² ont été mis en culture à l'interface air-liquide pendant 14 jours. Cinq mg/cm2 de pré-formules contenant les différents actifs ont ensuite appliqués à leur surface de manière homogène à l'aide d'un pinceau. Des triplicats ont été réalisés pour une durée de traitement de 24 heures à 37°C. Les épidermes ont alors été partagés: une moitié a été congelée en Tissue Tek puis des cryocoupes de 6 µm d'épaisseur ont été réalisées pour l'immunohistologie ; l'autre moitié a été congelée à sec à -80°C pour la préparation d'extraits protéiques totaux avant une analyse par Western. Les protéines totales ont été extraites par ébullition en tampon échantillon (0.175 M Tris-HCl pH 6,8 ; 12,5% bêta-mercaptoethanol, 7,5% SDS, 25% glycérol) et séparées par SDS-PAGE, en gels gradient de 4-15%.

### 2. Détection des protéines désiminées par immunohistologie

Les protéines désiminées ont été immunodétectées comme décrit précédemment après incubation dans le tampon de modification. Des contrôles négatifs ont été réalisés systématiquement en omettant la modification. Après lavage à l'eau, les coupes ont été incubées avec l'anticorps anti-citrulline AMC dilué au 1/1000^{ème}. Finalement une contre-coloration a été réalisée avec de l'hématoxyline.

### 3. Détection des protéines désiminées par Western

Les extraits protéiques, clarifiés par centrifugation et , séparés par PAGE-SDS ont été immunodétectés avec l'anticorps anti-citrulline AMC comme décrit plus haut ou avec un anticorps anti-actine (Millipore, clone MAB1501). Les intensités d'immunodétection ont été quantifiées comme décrit précédemment.

### Résultats :

### 1. Augmentation des protéines désiminées dans les épidermes reconstruits traités avec de l'acéfylline sous forme de sel de triéthanolamine ou de sel de sodium.

Des protéines désiminées avec une masse molaire apparente comprise entre 90 et 30 kDa sont détectées dans tous les extraits (voir Figure 9A). Une d'entre elles est la filaggrine (flèche). L'intensité d'immunodétection est plus importante (environ 2,5 fois) dans les pistes correspondant aux épidermes traités avec un gel aqueux contenant de l'acéfylline sous forme de sel de triéthanolamine ou de sel de sodium que dans celles correspondant aux épidermes traités avec le gel aqueux seul (voir Figures 9A et 9B).

### 2. Augmentation des protéines désiminées dans la couche cornée des épidermes reconstruits traités avec de l'acéfylline sous forme de sel de sodium.

Tous les échantillons qui n'ont pas subi la modification chimique sont négatifs (voir Figures 10D, 10E et 10F). Les échantillons d'épiderme reconstruits présentent avec l'anticorps AMC un marquage discontinu tout au long de la couche cornée. L'intensité d'immunodétection est plus forte après traitement avec l'acéfylline sous forme de sel de sodium (voir Figure 10).

En conclusion, l'acéfylline sous forme d'un de ses sels appliquée dans un gel aqueux à la surface des épidermes reconstruits augmente la désimination des protéines de la couche cornée et en particulier de la filaggrine.

### E. Exemple de préparation d'un gel aqueux contenant 3% d'acéfylline sous forme de sel de sodium

| **Nom INCI** | **Fonction** | **%** |
|---|---|---|
| Acefylline | Actif | 3 |
| Sodium Hydroxide | Base | 0.51 |
| Hydroxyethycellulose | Gélifiant | 1.5 |
| Phenoxyethanol | Conservateur | 0.7 |
| Aqua (Water) | Diluant | 94.29 |
| | | Ajout 2% eau |

### Mode opératoire

Etape A : Dans l'eau préalablement chauffée à 70°C, disperser l'acéfylline sous agitation (1300 tour/min) + ajouter NaOH : 70°C
Etape B : Ajouter le phénoxyethanol + saupoudrer lentement le natrosol en pluie sous agitation (500 tours/min) puis augmenter le cisaillement.
Puis B dans A sous agitation (1300 tours/min pendant 10 minutes). Refroidir et Homogénéiser (1h à 900 tours/min) pH final = 7,16

Observations macroscopiques : gel épais, transparent, incolore.

### EXEMPLES DE COMPOSITION

Plusieurs formulations sous forme de crème, ont les compositions suivantes (les quantités sont données en pourcentage massique par rapport au poids total de la composition).

Avantageusement, les formulations présentent entre 0,5 et 3% d'acéfylline ou un de ses sels, le pourcentage étant ajusté en fonction du pouvoir hydratant voulu.

**Exemple 1. :**

| Désignation | % massique | Fonction |
|---|---|---|
| acéfylline | 0,5 à 3 | Agent hydratant |
| Glycérol | 15 | Humectant |
| Vaseline | 8 | Emollient |
| Paraffine liquide | 2 | Emollient |
| acide stéarique | 1,5 | Emulsionnant |
| monostéarate de glycérol | 5 | Emulsionnant |
| cyclométhicone | 1,5 | Emollient |
| diméthicone | 0,5 | Emollient |
| polyéthylène glycol 600 | 5,0 | Humectant |
| triéthanolamine | QS | Agent neutralisant pH = 6,5 |
| Parahydroxybenzoate de propyle | QS | Conservateur |
| Eau | QSP 100,0 | |

**Exemple 2. :**

| Désignation | % massique | Fonction |
|---|---|---|
| acéfylline | 0,1 à 1 | Agent Hydratant |
| Huile Végétale | 1,5 | Emollient |
| Cétéaryl Glucoside/Cétéaryl alcohol | 5,0 | Emulsionnant |
| Stéarate de Glycéryle/PEG-100 stéarate | 2,5 | Emulsionnant |
| Paraffine liquide | 10,0 | Emollient |
| Beurre de karité | 1,0 | Emollient |
| PPG-15 STEARYL ETHER | 6,5 | Emollient |
| Cyclomethicone | 6,0 | Emollient |
| Glycérine 99.5% | 7,0 | Humectant |
| Gomme Xanthane | 0,1 | Gélifiant et stabilisant |
| Polyacrylamide | 0,8 | Gélifiant et stabilisant |
| Conservateurs | QS | Conservateur |
| Butylhydroxytoluene | 0,01 | Antioxydant |
| NaOH | QS | Agent neutralisant pH = 6,5 |
| Parfum | QS | Parfum |
| Eau | QSP 100,0 | |

## Revendications

1. Utilisation cosmétique d'une composition comprenant de l'acéfylline ou un de ses sels comme agent favorisant l'hydratation de la couche cornée de la peau.

2. Utilisation selon la revendication 1, **caractérisée en ce que** la composition est destinée à favoriser la désimination de la filaggrine dans l'épiderme.

3. Utilisation selon la revendication 1, **caractérisée en ce que** la composition est destinée à favoriser la production du FNH (facteur naturel d'hydratation) dans la couche cornée.

4. Utilisation selon la revendication 1, pour améliorer toute forme de sécheresse cutanée, ou pour renforcer les fonctions de barrière de l'épiderme et prévenir les signes de vieillissement cutané.

5. Utilisation selon la revendication 1 **caractérisée en ce que** la composition est destinée à favoriser la production naturelle d'acide transurocanique.

6. Utilisation selon la revendication 1, **caractérisée en ce que** l'acéfylline ou un de ses sels est associée à la caféine et/ou à la théobromine.

7. Procédé cosmétique pour hydrater la couche cornée de la peau comprenant l'application sur la peau d'une composition cosmétique comprenant de l'acéfylline ou un de ses sels.

8. Composition cosmétique contenant au moins un composé activateur de PAD1 et/ou PAD3 constitué par de l'acéfylline ou un de ses sels en association avec de la caféine et/ou de la théobromine et au moins un excipient cosmétiquement acceptable.

9. Composition cosmétique selon la revendication 8, **caractérisée en ce que** l'acéfylline ou un de ses sels représente entre 0,1% et 1% en poids, du poids total de la composition.

10. Composition selon l'une des revendications 8 et 9, **caractérisée en ce qu'**elle comprend au moins une phase grasse liquide comprenant au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange.

11. Composition selon l'une des revendications 8 à 10, **caractérisée en ce qu'**elle se présente sous la forme d'un produit de soin et/ou de maquillage de la peau du corps ou du visage, des lèvres, des cils, des sourcils, des cheveux, du cuir chevelu ou des ongles; d'un produit solaire ou autobronzant; d'un produit capillaire notamment de coloration, de conditionnement et/ou de soin des cheveux.

## Patentansprüche

1. Kosmetische Anwendung einer Zusammensetzung, umfassend Acefyllin oder eines seiner Salze als Wirkstoff, welcher die Feuchtigkeitsversorgung der Hornschicht der Haut fördert.

2. Anwendung nach Anspruch 1, **dadurch gekennzeichnet, dass** die Zusammensetzung zur Förderung der Deiminierung des Filaggrins in der Epidermis bestimmt ist.

3. Anwendung nach Anspruch 1, **dadurch gekennzeichnet, dass** die Zusammensetzung zur Förderung der Produktion des NMF (Natural Moisturizing Factor) in der Hornschicht bestimmt ist.

4. Anwendung nach Anspruch 1 zur Verbesserung jeder Form der Hauttrockenheit oder zur Stärkung der Barrierefunktionen der Epidermis und Vorbeugung der Zeichen von Hautalterung.

5. Anwendung nach Anspruch 1, **dadurch gekennzeichnet, dass** die Zusammensetzung zur Förderung der natürlichen Produktion von Transurocaninsäure bestimmt ist.

6. Anwendung nach Anspruch 1, **dadurch gekennzeichnet, dass** das Acefyllin oder eines seiner Salze mit dem Koffein und/oder mit dem Theobromin kombiniert ist.

7. Kosmetisches Verfahren zur Feuchtigkeitsversorgung der Hornschicht der Haut, umfassend das Auftragen auf die Haut einer kosmetischen Zusammensetzung, die Acefyllin oder eines seiner Salze umfasst.

8. Kosmetische Zusammensetzung, enthaltend mindestens eine Verbindung, die PAD1 und/oder PAD3 aktiviert, gebildet von Acefyllin oder einem seiner Salze in Kombination mit Koffein und/oder Theobromin, und mindestens einen kosmetisch akzeptablen Hilfsstoff.

9. Kosmetische Zusammensetzung nach Anspruch 8, **dadurch gekennzeichnet, dass** das Acefyllin oder eines seiner Salze zwischen 0,1 und 1 Gew.-% des Gesamtgewichts der Zusammensetzung darstellt.

10. Zusammensetzung nach einem der Ansprüche 8 und 9, **dadurch gekennzeichnet, dass** sie mindestens eine flüssige Fettphase umfasst, umfassend mindestens eine Verbindung, die aus den mineralischen, tierischen, pflanzlichen oder synthetischen, kohlenstoffhaltigen, kohlenwasserstoffhaltigen, fluorierten und/oder silikonierten, flüchtigen oder nicht flüchtigen Ölen und/oder Lösungsmitteln, allein oder im Gemisch, ausgewählt ist.

11. Zusammensetzung nach einem der Ansprüche 8 bis 10, **dadurch gekennzeichnet, dass** sie in Form eines Pflege- und/oder Make-up-Produkts für die Haut des Körpers oder des Gesichts, der Lippen, der Wimpern, der Augenbrauen, des Haars, der Kopfhaut oder der Nägel, eines Sonnenprodukts oder Selbstbräuners, eines Haarprodukts, insbesondere zum Färben, Konditionieren und/oder Pflegen des Haars, vorliegt.

## Claims

1. Cosmetic use of a composition including acefylline or a salt thereof as a moisturizing agent for the horny layer of the skin.

2. Use according to claim 1, **characterized in that** the composition is intended to promote the deimination of filaggrin in the epidermis.

3. Use according to claim 1, **characterized in that** the composition is intended to promote the production of NMF (natural moisturizing factor) in the horny layer.

4. Use according to claim 1, to improve any form of dry skin, or to strengthen the epidermal barrier function and to prevent the signs of aging skin.

5. Use according to claim 1, **characterized in that** the composition is intended to promote the natural production of trans-urocanic acid.

6. Use according to claim 1, **characterized in that** acefylline or a salt thereof is combined with caffeine and/or theobromine.

7. Cosmetic method to moisturize the horny layer of the skin comprising the application on the skin of a cosmetic composition including acefylline or a salt thereof.

8. Cosmetic composition containing at least one PAD1 and/or PAD3 activator compound consisting of acefylline or a salt thereof in combination with caffeine and/or theobromine and at least one cosmetically acceptable excipient.

9. Cosmetic composition according to claim 8, **characterized in that** acefylline or a salt thereof represents between 0.1% and 1% by weight of the total weight of the composition.

10. Composition according to one of claims 8 and 9, **characterized in that** it comprises at least one liquid fatty phase including at least one compound selected from oils and/or solvents of mineral, animal, vegetable or synthetic origin, containing carbon, hydrocarbon, fluorine and/or silicone, volatile or nonvolatile, alone or in mixture.

11. Composition according to one of claims 8 to 10, **characterized in that** it is provided as a care and/or makeup product for the skin of the body or face, the lips, lashes, eyebrows, hair, scalp or nails; as a sun or self-tanning product; as a hair product notably for coloring, conditioning and/or care of the hair.
